# EUROPEAN PATENT SPECIFICATION

(11) **EP 3 546 575 B1**
(45) Date of publication and mention of the grant of the patent: **17.07.2024**
(21) Application number: 17874261.5
(22) Date of filing: 24.11.2017
(51) Int. Cl.: C12N 15/10, C12N 15/85, C12N 15/90, C12N 5/10, C12N 13/00, A01K 67/027

(54) **GENOME EDITING METHOD**
GENOMEDITIERUNGSVERFAHREN
PROCÉDÉ D'ÉDITION DU GÉNOME

(30) Priority: 28.11.2016 JP 2016229976
(43) Date of publication of application: 02.10.2019
(73) Proprietor: Osaka University, Suita-shi, Osaka 565-0871 (JP)
(72) Inventor: MASHIMO, Tomoji, Suita-shi Osaka 565-0871 (JP); MIYASAKA, Yoshiki, Suita-shi Osaka 565-0871 (JP)
(74) Representative: J A Kemp LLP
(86) International application number: PCT/JP2017/042256
(87) International publication number: WO 2018/097257

(56) References cited:
- WO-A1-2016/133165
- WO-A1-2017/173092
- WO-A1-2017/180694
- JP-A- 2015 070 825
- WANG WENBO ET AL: "Delivery of Cas9 Protein into Mouse Zygotes through a Series of Electroporation Dramatically Increases the Efficiency of Model Creation", JOURNAL OF GENETICS AND GENOMICS, ELSEVIER LTD, AMSTERDAM, NL, vol. 43, no. 5, 8 March 2016 (2016-03-08), pages 319 - 327, XP029560554, ISSN: 1673-8527, DOI: 10.1016/J.JGG.2016.02.004
- MASAKAZU HASHIMOTO ET AL: "Electroporation enables the efficient mRNA delivery into the mouse zygotes and facilitates CRISPR/Cas9-based genome editing", SCIENTIFIC REPORTS, vol. 5, 11 June 2015 (2015-06-11), pages 11315, XP055363788, DOI: 10.1038/srep11315
- TAKEHITO KANEKO ET AL: "Simple knockout by electroporation of engineered endonucleases into intact rat embryos", SCIENTIFIC REPORTS,, vol. 4, 1 October 2014 (2014-10-01), pages 1 - 5, XP002768158, DOI: 10.1038/SREP06382
- HIROMI MIURA ET AL: "CRISPR/Cas9-based generation of knockdown mice by intronic insertion of artificial microRNA using longer single-stranded DNA", SCIENTIFIC REPORTS, vol. 5, 5 August 2015 (2015-08-05), pages 12799, XP055333089, DOI: 10.1038/srep12799
- KAZUTO YOSHIMI ET AL: "ssODN-mediated knock-in with CRISPR-Cas for large genomic regions in zygotes", NATURE COMMUNICATIONS, vol. 7, 20 January 2016 (2016-01-20), pages 10431, XP055361121, DOI: 10.1038/ncomms10431
- REMY SÉVERINE ET AL: "Generation of gene-edited rats by delivery of CRISPR/Cas9 protein and donor DNA into intact zygotes using electroporation", SCIENTIFIC REPORTS, vol. 7, no. 1, 1 December 2017 (2017-12-01), XP055853902, Retrieved from the Internet <URL:https://www.nature.com/articles/s41598-017-16328-y.pdf> DOI: 10.1038/s41598-017-16328-y
- NAKAGAWA, Y. ET AL.: "Ultra-superovulation for the CRISPR-Cas9-mediated production of gene -knockout, single-amino-acid-substituted, and floxed mice", BIOLOGY OPEN, vol. 5, 15 August 2016 (2016-08-15), pages 1142 - 1148, XP055516911, doi:10.1242/bio.019349
- RAN, F. A. ET AL.: "Double nicking by RNA-guided CRISPR Cas9 for enhanced genome editing specificity", CELL, vol. 154, 2013, pages 1380 - 1389, XP055299681, doi:10.1016/j.cell.2013.08.021
- DATABASE Nucleotide [online] 11 April 2007 (2007-04-11), STRAUSBERG, R. L. ET AL.: "Homo sapiens empty spiracles homeobox 1, mRNA (cDNA clone MGC:48662 IMAGE: 5260039), complete cds", XP055605166, Database accession no. BC045762.1
- YOSHIMI, K. ET AL.: "ssODN-mediated knock-in with CRISPR-Cas for large genomic regions in zygotes", NATURE COMMUNICATIONS, vol. 7, 20 January 2016 (2016-01-20), pages 10431, XP055361121
- MA, Y. ET AL.: "Generating rats with conditional alleles using CRISPR/Cas9", CELL RESEARCH, vol. 24, no. 1, January 2014 (2014-01-01), pages 122 - 125, XP055605167
- YANG, H. ET AL.: "One-step generation of mice carrying reporter and conditional alleles by CRISPR/Cas-mediated genome engineering", CELL, vol. 154, 2013, pages 1370 - 1379, XP028716273, doi:10.1016/j.cell.2013.08.022
- MASHIMO, T.: "Animal models of human diseases created by genome editing technology", CONGENITAL ANOMALIES, vol. 57, no. 6, August 2017 (2017-08-01), pages A1, XP009515824, DOI: 10.1111/cga.12253

## Description

### [Technical Field]

The present invention relates to an *in vitro* method for producing a genome edited cell, a genome editing composition, and a genome editing kit.

### [Background Art]

Genetically engineered mice are used by researchers around the world to analyze gene functions at an individual level. In particular, conditional knock-out mice are one of the most used genetically modified mice because they enable control of gene expression in a cell tissue specific and time specific manner.

Conditional knock-out mice are produced by crossing "Cre mice" incorporated with a Cre expression cassette and "flox mice" in which a target gene or a part thereof is sandwiched by loxP sequences . For the production of these Cre mice and flox mice, mouse ES cells have been mainly used conventionally, or some of them or Cre expression cassettes are knocked in to ES cells by homologous recombination. After that, the resulting cells are injected into mouse blastocysts, the embryos are implanted into pseudopregnant female mice, and chimeric mice are undergone to produce Cre mice and flox mice. Note that, since homologous recombination in ES cells requires skilled knowledge and technique, it takes several months even to establish recombinant ES cells alone. In addition, since chimeric mice are undergone, it takes one to two years to finally produce conditional mice.

In recent years, with the advent of a new genome editing technique, the CRISPR/Cas systems, it has become possible to more easily modify genes in fertilized eggs without using ES cells. When Cas mRNA and guide RNA are injected into a fertilized egg, the guide RNA binds to the target site, and the Cas protein induced at the binding site performs double-strand cleavage of DNA, making it possible as a result to introduce a mutation into the target gene. Here, when a single-stranded oligo (ssODN) is injected together, it is also possible to efficiently knock in a DNA sequence having a length of one to several tens of bases (NPL 1).

Meanwhile, in the case of producing a flox mouse with a CRISPR/Cas system, it is efficient if loxP sequences can be knocked in simultaneously at two sites, but in practice it often happens that a loxP sequence is knocked in only at one site. In this case, it is necessary to increase the production of mice in which a loxP sequence is knocked in at only one site, to introduce the CRISPR/Cas system to fertilized eggs collected from these knock-in mouse, and to knock in the loxP sequence at another site, which takes about another one year.

It is theoretically possible to simultaneously insert loxP sequences at two sites using a conventional donor vector, but in practice the efficiency with which flox mice are obtained is not high. Also reported are: a method for transferring Cas9 mRNA into mammalian fertilized egg by electroporation (PL 1); methods for genome editing in zygotes (PL 2); mammalian gene modification method using electroporation (PL 3); delivery of Cas9 protein into mouse zygotes through a series of electroporation dramatically increases the efficiency of model creation (NPL 2); electroporation enabling the efficient mRNA delivery into the mouse zygotes and facilitating CRISPR/Cas9-based genome editing (NPL 3) ; simple knockout by electroporation of engineered endonucleases into intact rat embryos (NPL 4); ultra-superovulation for the CRISPR-Cas9-mediated production of gene-knockout, single-amino-acid-substituted, and flexed mice (NPL 5); and, ssODN-mediated knock-in with CRISPR-Cas for large genomic regions in zygotes (NPL 6).

### [Citation List]

### [Patent Literature]

[PL 1] WO 2016/133165 A1
[PL 2] WO 2017/173092 A1
[PL 3] JP 2015-070825 A

### [Non Patent Literature]

[NPL 1] Sander JD, Joung JK. 'CRISPR-Cas systems for editing, regulating and targeting genomes.', Nat Biotechnol. 2014 Apr; 32(4): 347-55.
[NPL 2] WANG WENBO et al., 'Delivery of Cas9 Protein into Mouse Zygotes through a Series of Electroporation Dramatically Increases the Efficiency of Model Creation', JOURNAL OF GENETICS AND GENOMICS, 2016 Mar, 43(5): 319-327.
[NPL 3] MASAKAZU HASHIMOTO et al., 'Electroporation enables the efficient mRNA delivery into the mouse zygotes and facilitates CRISPR/Cas9-based genome editing', SCIENTIFIC REPORTS, 2015 Jun, 5: 11315.
[NPL 4] TAKEHITO KANEKO et al., 'Simple knockout by electroporation of engineered endonucleases into intact rat embryos', SCIENTIFIC REPORTS, 2014 Oct, 4: 1-5.
[NPL 5] NAKAGAWA, Y. et al., 'Ultra-superovulation for the CRISPR-Cas9-mediated production of gene-knockout, single-amino-acid-substituted, and flexed mice', Biology Open, 2016 Aug, 5: 1142-1148.
[NPL 6] YOSHIMI, K. etal., 'ssODN-mediated knock-in with CRISPR-Cas for large genomic regions in zygotes', Nature Communications, 2016 Jan, 7:10431.

### [Summary of Invention]

### [Technical Problem]

Described herein is a novel *in vitro* genome editing method which enables easier and more efficient introduction of even a mutation in a relatively long region or mutations at multiple sites in a relatively long region (for example, insertion of two loxP sequences).

### [Solution to Problem]

The present inventors have made earnest studies in light of the above object and have found as a result that it is possible to easily and efficiently perform genome editing by using two elements, namely (a) an artificial nuclease system which cleaves both ends of a genome editing target region, and (b) a nucleic acid sequence formed by arranging a 5'-side homology arm sequence, a donor DNA sequence, and a 3'-side homology arm sequence in this order from a 5'-side, the 5'-side homology arm sequence being a homologous sequence of one nucleic acid sequence outside the genome editing target region, and the 3'-side homology arm sequence being a homologous sequence of the other nucleic acid sequence outside the genome editing target region.

By using, in particular, a CRISPR/Cas system as an artificial nuclease system and single-stranded DNA whose donor DNA sequence contains two recombinant enzyme recognition sequences and introducing them into the target by the electroporation method, individuals were obtained with high efficiency added with the recombinant enzyme recognition sequences at both ends of the genome editing target region of each of the two alleles. In addition, by using fertilized eggs containing a recombinant enzyme expression cassette as targets for introduction of the artificial nuclease system, it was possible to significantly reduce the production period of the intended individuals.

Moreover, the present inventors have found that it is possible to enhance the above efficiency by employing a relatively long sequence as the 5'-side homology arm in single-stranded DNA. Those findings have led to the completion of the present invention.

The invention is defined in the independent claims. Further aspects and preferred embodiments are defined in the dependent claims. Any aspects, embodiments and examples of the present disclosure which do not fall under the scope of the appended claims do not form part of the invention and are merely provided for illustrative purposes.

Specifically, the present invention provides an *in vitro* method for producing a genome edited cell, comprising the step of introducing
(a) an artificial nuclease system which cleaves both ends of a genome editing target region, and
(b) a single-stranded DNA which contains a nucleic acid sequence comprising a 5'-side homology arm sequence, a donor DNA sequence, and a 3'-side homology arm sequence in this order from a 5'-side, the 5'-side homology arm sequence being a homologous sequence of one nucleic acid sequence outside the genome editing target region and 110 bases long or more, the donor DNA sequence being 200 bases long or more and 800 bases long or less, and the 3'-side homology arm sequence being a homologous sequence of the other nucleic acid sequence outside the genome editing target region, into a cell by an electroporation method, wherein the cell is not a human zygote.

The present invention also provides a genome editing composition for producing a genome edited cell or a genome edited organism, comprising:
(a) an artificial nuclease system which cleaves both ends of a genome editing target region; and
(b) a single-stranded DNA which contains a nucleic acid sequence comprising a 5'-side homology arm sequence, a donor DNA sequence, and a 3'-side homology arm sequence in this order from a 5'-side, the 5'-side homology arm sequence being a homologous sequence of one nucleic acid sequence outside the genome editing target region and 110 bases long or more, the donor DNA sequence being 200 bases long or more and 800 bases long or less, and the 3'-side homology arm sequence being a homologous sequence of the other nucleic acid sequence outside the genome editing target region.

The present invention also provides a genome editing kit for producing a genome edited cell or a genome edited organism, comprising:
(a) an artificial nuclease system which cleaves both ends of a genome editing target region; and
(b) a single-stranded DNA which contains a nucleic acid sequence comprising a 5'-side homology arm sequence, a donor DNA sequence, and a 3'-side homology arm sequence in this order from a 5'-side, the 5'-side homology arm sequence being a homologous sequence of one nucleic acid sequence outside the genome editing target region and 110 bases long or more, the donor DNA sequence being 200 bases long or more and 800 bases long or less, and the 3'-side homology arm sequence being a homologous sequence of the other nucleic acid sequence outside the genome editing target region.

### [Advantageous Effects of Invention]

The present invention makes it possible to more easily and efficiently replace a genome editing target region with any donor DNA sequence. This enables easier and more efficient introduction of even a mutation in a relatively long region or mutations at multiple sites in a relatively long region (for example, insertion of two loxP sequences), which is difficult in conventional methods (such as NPL 1) using a relatively short single-stranded oligo (ssODN).

In addition, while conventional methods such as methods using ES cells and methods using a relatively short single-stranded oligo (ssODN) (such as NPL 1) take at least one year to produce conditional knock-out mice, use of the *in vitro* genome editing method described herein makes it possible to produce conditional knock-out mice in a shorter period of time (for example, three to six months, and even a shorter period of time in the case of using Cre mouse fertilized eggs). Along with this, it is also possible to significantly reduce the production cost of conditional knock-out mice (as an estimation example, conventional method: 3,000,000 to 9, 000, 000 yen/strain → the *in* vitro method described herein: 500, 000 to 1, 000, 000 yen/strain) .

Moreover, in the *in vitro* method described herein, it is also possible to introduce various tools for genome editing target cells (the above materials (a) and (b)) by the electroporation method. The microinjection method is usually employed conventionally, but when this electroporation method is employed, it is possible to more easily and efficiently perform genome editing.

Currently, a comprehensive knock-out mouse production project for about 30,000 genes is in progress, and the present invention can accelerate the progress of such a project.

### [Brief Description of Drawings]

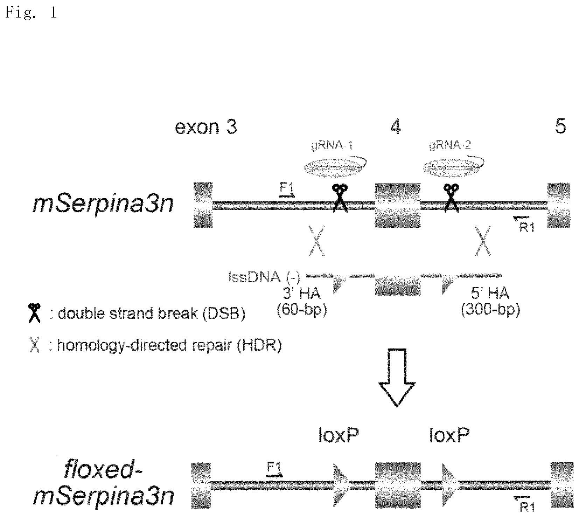

[Fig. 1] Fig. 1 illustrates a scheme of Genome Editing Test 1 (Example 1). The mSerpina3n represents genomic DNA before genome editing, lssDNA represents single-stranded DNA, and floxed-mSerpina3n represents genomic DNA after genome editing. The scissors symbol indicates the site to be cleaved by the CRISPR/Cas system (the region sandwiched between the two scissors symbols is the genome editing target region), gRNA-1 and gRNA-2 represent two guide RNAs designed to cleave both ends of the genome editing target region, the arrows (F1 and R1) indicate the positions of PCR primers used for genotyping, the directions of the arrows indicate the directions of the primers (5' → 3'), (-) indicates that single-stranded DNA is an antisense strand, 5' HA represents a 5' homology arm (base length in parentheses), 3' HA represents a 3' homology arm (base length in parentheses), the triangles represent loxP sequences, the orientations of the triangles in the horizontal direction indicate the orientations of the loxP sequences, and the × between mSerpina3n and lssDNA indicates that the region is a homologous sequence.

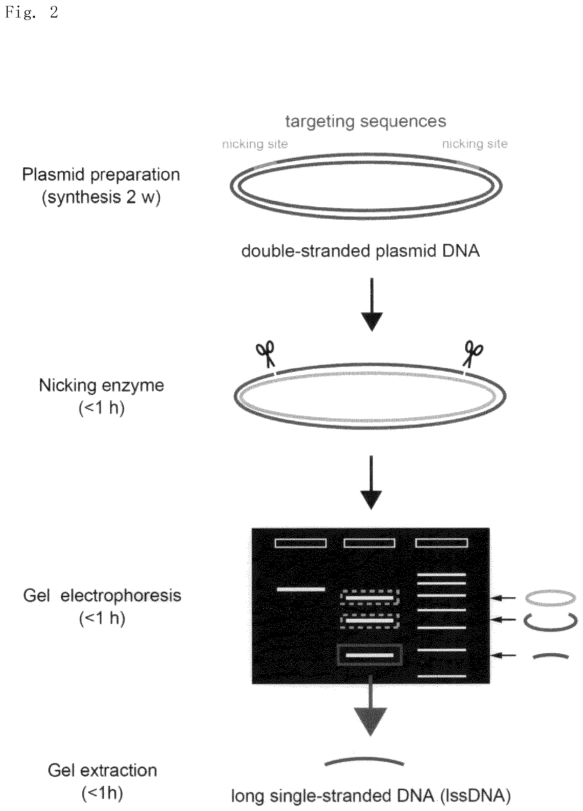

[Fig. 2] Fig. 2 illustrates an overview of the method for producing a single-stranded DNA, which includes first preparing a plasmid DNA containing nicking endonuclease recognition sites in "Plasmid preparation," digesting the plasmid DNA using nicking endonucleases in "Nicking enzyme," subjecting the digested product to agarose gel electrophoresis in "Gel electrophoresis," and extracting single-stranded DNA from gel containing a band matching the intended single-stranded DNA in "Gel extraction."

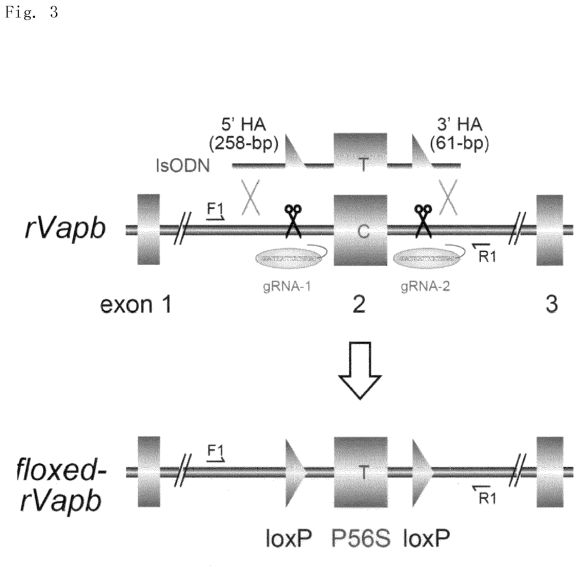

[Fig. 3] Fig. 3 illustrates a scheme of Genome Editing Test 5 (Example 5) . The rVapb represents genomic DNA before genome editing, lsODN represents single-stranded DNA, and floxed-rVapb represents genomic DNA after genome editing. The scissors symbol indicates the site to be cleaved by the CRISPR/Cas system (the region sandwiched between the two scissors symbols is the genome editing target region), gRNA-1 and gRNA-2 represent two guide RNAs designed to cleave both ends of the genome editing target region, the arrows (F1 and R1) indicate the positions of PCR primers used for genotyping, the directions of the arrows indicate the directions of the primers (5' → 3'), 5' HA represents a 5' homology arm (base length in parentheses), 3' HA represents a 3' homology arm (base length in parentheses), the triangles represent loxP sequences, the orientations of the triangles in the horizontal direction indicate the orientations of the loxP sequences, the × between rVapb and lsODN indicates that the region is a homologous sequence, T or C in exon 4 indicates a base to be mutated by genome editing, and P56S indicates that the mutation causes mutation of the 56th amino acid of rVapb protein (proline) to serine.

### [Description of Embodiments]

### 1. Definitions

In the present specification, the expressions "contain" and "include" include the concepts of "contain," "include," "composed essentially of," and "composed only of."

The "identity" of amino acid sequences refers to the degree to which two or more comparable amino acid sequences match each other. Therefore, the higher the matching degree between certain two amino acid sequences, the higher the identity or similarity of those sequences. The level of the identity of amino acid sequences is determined using, for example, FASTA, a tool for sequence analysis, and using default parameters. Alternatively, determination is possible using the algorithm BLAST by Karlin and Altschul (KarlinS, Altschul SF. "Methods for assessing the statistical significance of molecular sequence features by using general scoring schemes "Proc Natl Acad Sci USA. 87: 2264-2268 (1990), KarlinS, Altschul SF. "Applications and statistics for multiple high-scoring segments in molecular sequences." Proc Natl Acad Sci USA. 90: 5873-7 (1993)). A program called BLASTX based on such BLAST algorithm has been developed. The specific procedures for these analysis methods are known, and one may refer to the website of the National Center of Biotechnology Information (NCBI)
(http://www.ncbi.nlm.nih.gov/). In addition, the "identity" of nucleic acid sequences is also defined in the same manner as described above.

In the present specification, the "conservative substitution" means that an amino acid residue is substituted with an amino acid residue having a similar side chain. For example, substitution with amino acid residues having basic side chains such as lysine, arginine, and histidine corresponds to the conservative substitution. In addition, similarly, the conservative substitution also includes substitution with amino acid residues having acidic side chains such as aspartic acid and glutamic acid; amino acid residues having uncharged polar side chains such as glycine, asparagine, glutamine, serine, threonine, tyrosine, and cysteine; amino acid residues having nonpolar side chains such as alanine, valine, leucine, isoleucine, proline, phenylalanine, methionine, and tryptophan; amino acid residues having β-branched side chains such as threonine, valine, and isoleucine; and amino acid residues having aromatic side chains such as tyrosine, phenylalanine, tryptophan, and histidine.

In the present specification, nucleotides such as DNA and RNA used exogenously may be subjected to known chemical modifications as exemplified below. For the purpose of preventing degradation due to hydrolytic enzymes such as nucleases, the phosphate residues (phosphates) of the nucleotides can be substituted with, for example, chemically modified phosphate residues such as phosphorothioate (PS), methyl phosphonate, and phosphorodithionate. In addition, the hydroxyl group at position 2 of the sugar (ribose) of each ribonucleotide may be substituted with -OR (R represents, for example, CH3 (2'-O-Me), CH2CH2OCH3 (2'-O-MOE), CH2CH2NHC(NH)NH2, CH2CONHCH3, CH2CH2CN, and the like) . Moreover, the base moiety (pyrimidine, purine) may be chemically modified, and examples thereof include introduction of a methyl group or a cationic functional group into position 5 of a pyrimidine base and substitution of a carbonyl group at position 2 with a thiocarbonyl. Further examples include, but are not limited to, ones in which the phosphate moiety or the hydroxyl moiety is modified with, for example, biotin, an amino group, a lower alkylamine group, an acetyl group, or the like. In addition, preferably used examples also include BNA (LNA) in which the 2' oxygen and the 4' carbon of the sugar moiety of the nucleotide are cross-linked to fix the conformation of the sugar moiety to the N-type.

### 2. Materials

The present invention relates in an aspect thereof to an in *vitro* method for producing a genome edited cell, a genome editing composition, and a genome editing kit, which use two types of materials (material (a) and material (b)). Hereinafter, these materials are described.

### 2-1. Material (a)

The material (a) is an artificial nuclease system which cleaves both ends of a genome editing target region.

The genome editing target region is any region on genomic DNA of a genome editing target cell or organism, and is a target region to be replaced with the donor DNA sequence to be described later by genome editing.

The length of the genome editing target region is not particularly limited. The length is, for example, 10 bases long or more, preferably 30 bases long or more, more preferably 60 bases long or more, further preferably 100 bases long or more, even further preferably 150 bases long or more, and even further preferably 200 bases long or more, and, for example, 10000 bases long or less, preferably 5000 bases long or less, more preferably 2000 bases long or less, further preferably 1000 bases long or less, and even further preferably 800 bases long or less.

The genome editing target cell is not particularly limited as long as it is not a human zygote and it can be genome edited with an artificial nuclease system. Examples of the genome editing target cell include cells derived from various tissues or cells of various properties, such as blood cells, hematopoietic stem cells/progenitor cells, gametes (sperms, ova), fibroblasts, epithelial cells, vascular endothelial cells, neurons, hepatocytes, keratinocytes, myocytes, epidermal cells, endocrine cells, ES cells, iPS cells, tissue stem cells, and cancer cells. Examples of the genome editing target organisms include animals such as mammalian animals such as monkeys, mice, rats, guinea pigs, hamsters, pigs, cattle, goats, sheep, dogs, cats, and rabbits; amphibians such as African clawed frogs; fish animals such as zebrafish, Japanese rice fish, Japanese pufferfish, tuna, and red seabream; chordates such as sea squirts; arthropods such as vinegar flies and silkworms: plants such as thale cress, tomato, soybean, rice, wheat, corn, rapeseed, and tobacco: fungi such as yeast and red bread mold: and bacteria such as Escherichia coli, hay bacillus, and blue-green algae.

The mammalian animals are preferably rodent animals such as mice, rats, guinea pigs, and hamsters, and particularly preferably mice or rats.

In addition, if it is intended to produce a condition specific gene mutant (for example, a conditional knock-out creature) or the like using a recombinant enzyme and its recognition sequence (for example, Cre-loxP system or FLP-FRT system), it is possible to extremely easily and efficiently obtain a condition specific gene mutant by using a fertilized egg containing a recombinant enzyme expression cassette (for example, a fertilized egg fertilized with a sperm containing a Cre or FLP expression cassette) as the fertilized egg and using a donor DNA containing two recombinant enzyme recognition sequences (for example, loxP sequence and FRT sequence) as the donor DNA to be described later.

The recombinant enzyme expression cassette is not particularly limited as long as it is a DNA capable of expressing the recombinant enzyme in cells. Typical examples of the recombinant enzyme expression cassette include a promoter and a DNA containing a recombinant enzyme coding sequence arranged under the control of the promoter. The promoter for the expression cassette is not particularly limited, and it is possible to widely employ a promoter whose transcriptional activity changes depending on various conditions, such as a tissue specific promoter, a time specific promoter, a and drug responsive promoter.

The cleavage at both ends of a genome editing target region is, in other words, the cleavage at two sites of one end and the other end of the genome editing target region. More specifically, it is the cleavage between the base pair at one end of the genome editing target region and the adjacent base pair outside that base pair (outside the genome editing target region) and the cleavage between the base pair at the other end of the genome editing target region and the adjacent base pair outside that base pair (outside the genome editing target region).
Consequently, a DNA fragment containing the genome editing target region is generated from genomic DNA.

The artificial nuclease system is not particularly limited as long as it is a system capable of cleaving any two sites on the genomic DNA to generate a cleaved fragment. Examples of the artificial nuclease system include the CRISPR/Cas systems, the TALEN system, the ZFN system, and the PPR system.

A CRISPR/Cas system uses the Cas protein, which is a nuclease (RGN; RNA-guided nuclease), and a guide RNA (gRNA). When the system is introduced into a cell, the guide RNA binds to the target site, and the DNA can be cleaved with the Cas protein induced at the binding site.

The TALEN system uses an artificial nuclease (TALEN) which contains a DNA cleavage domain (for example, a FokI domain) as well as a DNA binding domain of a transcriptional activator-like (TAL) effector (for example, US Patent No. 8470973 and US Patent No. 8586363). When the system is introduced into a cell, TALEN binds to the target site via the DNA binding domain, where it cleaves the DNA. The DNA binding domain which binds to the target site can be designed in accordance with a known scheme (for example, Zhang, Feng et. al. (2011) Nature Biotechnology 29 (2)).

The ZFN system uses an artificial nuclease (ZFN) which contains a nucleic acid cleavage domain conjugated to a DNA binding domain containing a zinc finger array (for example, US Patent No. 6265196, 8524500, 7888121, and European Patent No. 1720995). When the system is introduced into a cell, ZFN binds to the target site via the DNA binding domain, where it cleaves the DNA. The DNA binding domain which binds to the target site can be designed in accordance with a known scheme.

As the PPR system, it is possible to use, for example, PPR (pentatricopeptide repeat) fused with a nuclease domain (Nakamura et al., Plant Cell Physiol 53: 1171-1179 (2012)).

The CRISPR/Cas systems are preferable among the artificial nuclease systems from the viewpoint that they use a nucleic acid (guide RNA) for the recognition of the target site and thus the target site can be selected more freely and its preparation is easy. A CRISPR/Cas system is typically composed of Cas components such as Cas protein, Cas mRNA, and a Cas protein expression cassette and guide RNA components such as guide RNA designed to cleave both ends of the genome editing target region and its expression cassette. Hereinafter, the components are described.

### 2-1-1. Cas Component (Material (a1))

Examples of the Cas component include Cas proteins, Cas mRNA, and Cas protein expression cassettes. The Cas components may be used singly or in combination of two or more kinds.

The Cas protein is not particularly limited as long as it is used in a CRISPR/Cas system, and it is possible to use, for example, various types which are capable of binding to the target site of genomic DNA while forming a complex with guide RNA and which are capable of performing double-strand cleavage of the target site.

The "target site" of the present specification is a site on genomic DNA composed of a DNA strand (target strand) composed of a PAM (Proto-spacer Adjacent Motif) sequence and a sequence of about 17 to 30 bases long (preferably 18 to 25 bases long, more preferably 19 to 22 bases long, and particularly preferably 20 bases long) adjacent to its 5'-side, and its complementary DNA strand (non-target strand).

Cas proteins derived from various organisms are known, and examples of the Cas proteins include Cas9 protein derived from S. pyogenes (type II), Cas9 protein derived from S. solfataricus (type I-A1), Cas9 protein derived from S. solfataricus (type I-A2), Cas9 protein derived from H. walsbyl (type I-B), Cas9 protein derived from E. coli (type I-E), Cas9 protein derived from E. coli (type I-F), Cas9 protein derived from P. aeruginosa (type I-F), Cas9 protein derived from S. Thermophilus (type II-A), Cas9 protein derived from S. agalactiae (type II-A), Cas9 protein derived from S. aureus, Cas9 protein derived from N. meningitidis, and Cas9 protein derived from T. denticola. Among these, a preferable example is Cas9 protein, and a more preferable example is Cas9 protein endogenously having bacteria belonging to the Streptococcus genus. In addition, it is also possible to use Cpf1 protein as a Cas protein. Preferable examples of the Cpf1 protein include Cpf1 protein derived from Lachnospiraceae bacterium or Acidaminococcus sp.

Information on the amino acid sequences of various Cas proteins and their coding sequences can be easily obtained from literatures and various databases such as NCBI (WO2014/131833 and accession numbers: Q99ZW2.1, WP_021736722, WP_035635841, and the like).

The PAM sequence differs depending on the type of Cas protein used. For example, the PAM sequence corresponding to the Cas9 protein derived from S. pyogenes (type II) is 5'-NGG, the PAM sequence corresponding to the Cas9 protein derived from S. solfataricus (type I-A1) is 5'-CCN, the PAM sequence corresponding to the Cas9 protein derived from S. solfataricus (type I-A2) is 5'-TCN, the PAM sequence corresponding to the Cas9 protein derived from H. walsbyl (type I-B) is 5'-TTC, the PAM sequence corresponding to the Cas9 protein derived from E. coli (type I-E) is 5'-AWG, the PAM sequence corresponding to the Cas9 protein derived from E. coli (type I-F) is 5'-CC, the PAM sequence corresponding to the Cas9 protein derived from P. aeruginosa (type I-F) is 5'-CC, the PAM sequence corresponding to the Cas9 protein derived from S. Thermophilus (type II-A) is 5'-NNAGAA, the PAM sequence corresponding to the Cas9 protein derived from S. agalactiae (type II-A) is 5'-NGG, the PAM sequence corresponding to the Cas9 protein derived from S. aureus is 5'-NGRRT or 5'-NGRRN, the PAM sequence corresponding to the Cas9 protein derived from N. meningitidis is 5'-NNNNGATT, and the PAM sequence corresponding to the Cas9 protein derived from T. denticola is 5'-NAAAAC. In addition, the PAM sequence of Cpf1 is, for example, 5'-TTN or 5'-TTTN.

Note that it is also possible to modify the PAM recognition by modifying the Cas protein (for example, introduction of a mutation) Benjamin, P. et al., Nature 523, 481-485 (2015), Hirano, S. et al., Molecular Cell 61, 886-894 (2016)). This makes it possible to expand the choice of target site.

The Cas protein may have a mutation as long as the nuclease activity is not impaired. From this viewpoint, the Cas protein may be a protein which is composed of an amino acid sequence having, for example, 85% or more, preferably 90% or more, more preferably 95% or more, and further preferably 98% or more identity with the amino acid sequence of the original wild type Cas protein, and which has its activity (activity of binding to the target site of genomic DNA while forming a complex with guide RNA and cleaving the target site) . Alternatively, from the same viewpoint, the Cas protein may be a protein which is composed of an amino acid sequence in which one or more (for example, 2 to 100, preferably 2 to 50, more preferably 2 to 20, further preferably 2 to 10, even further preferably 2 to 5, and particularly preferably 2) amino acids are substituted, deleted, added, or inserted into the amino acid sequence of the original wild type Cas protein, and which has its activity (activity of binding to the target site of genomic DNA while forming a complex with guide RNA and cleaving the target strand or the non-target strand of the target site). Note that the "activity" described above can be evaluated *in vitro* or in vivo in accordance with a known method or in a similar manner. Also, the mutation is preferably a conservative substitution.

As the Cas protein, it is also possible to use a Cas9 protein in which one of the catalytic sites is introduced with a mutation to exhibit a nickase activity (nCas protein). Use of an nCas protein makes it possible to reduce off-target effects.

The Cas protein may be one chemically modified as long as it has the "activity" described above.

In the Cas protein, the C-terminus may be any of a carboxyl group (-COOH), a carboxylate (-COO⁻), an amide (-CONH₂), and an ester (-COOR).

Here, R used in the ester is, for example, a C₁₋₆ alkyl group such as methyl, ethyl, n-propyl, isopropyl, or n-butyl; for example, a C₃₋₈ cycloalkyl group such as cyclopentyl or cyclohexyl; for example, a C₆₋₁₂ aryl group such as phenyl or α-naphthyl; for example, a phenyl-C₁₋₂ alkyl group such as benzyl or phenethyl; a C₇₋₁₄ aralkyl group such as an α-naphthyl-C₁₋₂ alkyl group including α-naphthylmethyl; or a pivaloyloxymethyl group.

In the Cas protein, a carboxyl group (or a carboxylate) other than the C-terminus may be amidated or esterified. As the ester in this case, for example, the above-described C-terminal ester or the like is used.

Moreover, the Cas protein includes one in which the amino group of the N-terminal amino acid residue is protected by a protecting group (for example, a formyl group or a C₁₋₆ acyl group such as a C₁₋₆ alkanoyl including an acetyl group), one in which the N-terminal glutamine residue, which can be cleaved and generated in vivo, has been pyroglutaminated, one in which the substituent on the side chain of the amino acid in the molecule (for example, -OH, -SH, amino group, imidazole group, indole group, guanidino group, or the like) is protected by a suitable protecting group (for example, a formyl group or a C₁₋₆ acyl group such as a C₁₋₆ alkanoyl group including an acetyl group), a complex protein such as a so-called glycoprotein bound with sugar chains, or the like.

The Cas protein may be one added with a known protein tag, a signal sequence, or a protein such as an enzyme protein as long as it has the "activity" described above. Examples of the protein tag include biotin, His tag, FLAG tag, Halo tag, MBP tag, HA tag, Myc tag, V5 tag, and PA tag. Examples of the signal sequence include nuclear localization signals. Examples of the enzyme protein include various histone modifying enzymes and deaminases.

The Cas protein may be in the form of a pharmaceutically acceptable salt with an acid or a base. The salt is not particularly limited as long as it is a pharmaceutically acceptable salt, and it is possible to employ both an acid salt and a basic salt. Examples of the acid salt include inorganic acid salts such as hydrochlorides, hydrobromides, sulfates, nitrates, and phosphates; organic salts such as acetates, propionates, tartrates, fumarates, maleates, malates, citrates, methanesulfonates, and para toluenesulfonates; and amino acid salts such as aspartates and glutamates. In addition, examples of the basic salt include alkali metal salts such as sodium salts and potassium salts; and alkaline earth metal salts such as calcium salts and magnesium salts.

The Cas protein may be in the form of a solvate. The solvent is not particularly limited as long as it is pharmaceutically acceptable, and examples thereof include water, ethanol, glycerol, and acetic acid.

The Cas mRNA is not particularly limited as long as it contains a Cas protein coding sequence and can express the Cas protein when translated in a genome editing target cell or in a cell of a genome editing target organism. In addition to the Cas protein coding sequence, the Cas mRNA can contain various sequences and various modification structures, for example, a 5' untranslated region having a 5' cap structure, a Shine-Dalgarno sequence, a Kozak sequence, IRES, and the like; and a 3' untranslated region having a polyadenylation signal, an AU rich element, a GU rich element, and the like.

The Cas protein expression cassette is not particularly limited as long as it is a DNA capable of expressing the Cas protein in a genome editing target cell or in a cell of a genome editing target organism. Typical examples of the Cas protein expression cassette include a promoter and a DNA containing a Cas protein coding sequence arranged under the control of the promoter.

The promoter contained in the Cas protein expression cassette is not particularly limited, and it is possible to use, for example, various types of pol II type promoters. Examples of the pol II type promoters include, but are not limited to, CMV promoters, EF1 promoters, SV40 promoters, MSCV promoters, hTERT promoters, β actin promoters, and CAG promoters.

The Cas protein expression cassette may be contained on the same DNA (one molecule of DNA) as the guide RNA expression cassette to be described later, or may be contained on separate DNAs (different DNA molecules).

The Cas protein expression cassette may optionally contain additional elements (for example, a multiple cloning site (MCS), a drug resistance gene, an origin of replication, and the like). In the case of arrangement of the promoter and the Cas protein coding sequence in this order from the 5'-side, an example of the Cas protein expression cassette is such that an MCS is arranged between the promoter and the Cas protein coding sequence (preferably adjacent to one of them or both of them) on (preferably adjacent to) the 3'-side of the Cas protein coding sequence. The MCS is not particularly limited as long as it contains multiple (for example, 2 to 50, preferably 2 to 20, and more preferably 2 to 10) restriction enzyme sites.

Examples of the drug resistance gene include chloramphenicol resistance genes, tetracycline resistance genes, neomycin resistance genes, erythromycin resistance genes, spectinomycin resistance genes, kanamycin resistance genes, hygromycin resistance genes, and puromycin resistance genes.

The Cas protein expression cassette may alone constitute a vector or may constitute a vector together with other sequences. The type of vector is not particularly limited, and examples thereof include plasmid vectors such as animal cell expression plasmids; virus vectors such as retroviruses, lentiviruses, adenoviruses, adeno-associated viruses, herpesviruses, and Sendai viruses; and Agrobacterium vectors.

The Cas components described above can be easily produced in accordance with known genetic engineering methods. For example, production is possible using PCR, restriction enzyme cleavage, DNA ligation technique, *in vitro* transcription/translation technique, recombinant protein production technique, and the like.

### 2-1-2. Guide RNA Component (Material (a2))

Examples of the guide RNA component include guide RNAs designed to cleave both ends of the genome editing target region and their expression cassettes. The guide RNA components may be used singly or in combination of two or more kinds.

The guide RNA is not particularly limited as long as it is used in a CRISPR/Cas system, and it is possible to use, for example, various types which are capable of binding to the target site of genomic DNA and binding to a Cas protein, making it possible to induce the Cas protein to the target site of the genomic DNA.

The guide RNA has a sequence involved in the binding to the target site of genomic DNA (sometimes referred to as a crRNA (CRISPR RNA) sequence), and when this crRNA sequence binds complementarily (preferably, in a complementary and specific manner) to a sequence obtained by excluding the PAM sequence complementary sequence of the non-target strand, the guide RNA can bind to a target site of genomic DNA.

Note that the phrase binds "complementarily" includes not only the case of binding based on perfect complementarity (A and T, and G and C) but also the case of binding based on complementarity to such a degree that allows hybridization under stringent conditions. The stringent conditions can be determined based on the melting temperature (Tm) of the nucleic acid which binds a probe or a complex, as taught in Berger and Kimmel (1987, Guide to Molecular Cloning Techniques Methods in Enzymology, Vol. 152, Academic Press, San Diego CA). Usually, example of the washing conditions after hybridization can include conditions of about "1 × SSC, 0.1% SDS, 37°C." It is preferable that the hybridization state be maintained even when washing is performed under such conditions. Although not particularly limited, examples of the washing conditions can include more strict hybridization conditions of about "0.5 × SSC, 0.1% SDS, 42°C" and further strict hybridization conditions of about "0.1 × SSC, 0.1% SDS, 65°C."

Specifically, among crRNA sequences, sequences which bind to the target sequence have, for example, 90% or more, preferably 95% or more, more preferably 98% or more, further preferably 99% or more, and particularly preferably 100% identity with the target strand. Note that, among the crRNA sequences, 12 bases on the 3'-side of the sequence which binds to the target sequence are said to be important for the binding of the guide RNA to the target site. For this reason, among the crRNA sequences, when the crRNA sequence which binds to the target sequence is not completely identical to the target strand, the bases different from those of the target strand are preferably present in the crRNA sequence other than at the 12 bases on the 3'-side of the sequence which binds to the target sequence.

For example, when the Cas protein is Cas9 protein, the guide RNA has a sequence involved in the binding to the Cas9 protein (sometimes referred to as tracrRNA (trans-activating crRNA) sequence), and when this tracrRNA sequence binds to the Cas9 protein, it can induce the Cas9 protein to the target site of genomic DNA.

The tracrRNA sequence is not particularly limited. The tracrRNA sequence is typically an RNA composed of a sequence of about 50 to 100 bases long capable of forming multiple (generally three) stem loops, and the sequence differs depending on the type of Cas protein used. As the tracrRNA sequence, it is possible to employ various known sequences depending on the type of Cas protein used.

An example of the guide RNA containing the crRNA sequence and the tracrRNA sequence may be a single-stranded RNA (sgRNA) containing the crRNA sequence and the tracrRNA sequence, or may be an RNA complex formed by complementarily binding an RNA containing the crRNA sequence and an RNA containing the tracrRNA sequence.

On the other hand, although the Cpf1 protein binds to crRNA, tracrRNA is unnecessary in the guide RNA. In addition, the Cpf1 protein differs in that it generates a protruding end whereas the Cas9 protein generates a blunt end as a result of cleaving the target double-stranded DNA.

The guide RNAs designed to cleave both ends of the genome editing target region are not particularly limited as long as they are a guide RNA whose target site is designed such that the Cas protein cleaves both ends of the genome editing target region, and can easily be designed based on the information described above. The cleavage of both ends of the region is performed by a guide RNA designed to cleave one end (gRNA 1) and a guide RNA designed to cleave the other end (gRNA 2). The sequence of the target site of gRNA 1 and the sequence of the target site of gRNA 2 may be the same, but they are preferably different from each other from the viewpoint that it is possible to further reduce the off-target effects.

Note that, in the case of using the nCas protein, it is possible to use, for each of the strands of the double-stranded DNA at the target site, a guide RNA targeting one site (two sites in total), for example.

The guide RNA expression cassette is not particularly limited as long as it is a DNA capable of expressing (transcribing) a guide RNA in a genome editing target cell or in a cell of a genome editing target organism. For example, when the guide RNA is a single-stranded RNA containing the crRNA sequence and the tracr RNA sequence, a typical example of the guide RNA expression cassette is a DNA having a promoter and a guide RNA coding sequence arranged under the control of the promoter. As another example, when the guide RNA is an RNA complex formed by complementarily binding an RNA containing the crRNA sequence and an RNA containing the tracrRNA sequence, a typical example of the guide RNA expression cassette is a combination of an expression cassette containing a promoter and a "crRNA sequence-containing RNA" coding sequence arranged under the control of the promoter (which may be expresses as the "crRNA expression cassette" for convenience) and an expression cassette containing a promoter and a "tracrRNA sequence-containing RNA" coding sequence arranged under the control of the promoter (which may be expresses as the "tracrRNA expression cassette" for convenience), or the like.

The promoter contained in the guide RNA expression cassette is not particularly limited, and it is possible to use pol II type promoters, but pol III type promoters are preferable from the viewpoint of more accurately transcribing relatively short RNA. Examples of the pol III type promoters include, but are not limited to, mouse and human U6-snRNA promoters, human H1-RNase P RNA promoters, and human valine-tRNA promoters.

When the guide RNA expression cassette is a combination of a crRNA expression cassette and a tracrRNA expression cassette, these two expression cassettes may be contained on the same DNA (one molecule of DNA), or they may be contained on separate DNAs (different DNA molecules).

The guide RNA expression cassette may be contained on the same DNA (one molecule of DNA) as the Cas protein expression cassette described above, or may be contained on separate DNAs (different DNA molecules).

The guide RNA expression cassette may optionally contain additional elements (for example, a multiple cloning site (MCS), a drug resistance gene, an origin of replication, and the like). In the case of arrangement of the promoter and the crRNA sequence coding sequence in this order from the 5'-side, an example of the guide RNA expression cassette is such that an MCS is arranged between the promoter and the crRNA sequence coding sequence (preferably adjacent to one of them or both of them) on (preferably adjacent to) the 3'-side of the crRNA sequence coding sequence. The MCS is not particularly limited as long as it contains multiple (for example, 2 to 50, preferably 2 to 20, and more preferably 2 to 10) restriction enzyme sites.

Examples of the drug resistance gene include chloramphenicol resistance genes, tetracycline resistance genes, neomycin resistance genes, erythromycin resistance genes, spectinomycin resistance genes, kanamycin resistance genes, hygromycin resistance genes, and puromycin resistance genes.

The guide RNA expression cassette may alone constitute a vector or may constitute a vector together with other sequences. The type of vector is not particularly limited, and examples thereof include plasmid vectors such as animal cell expression plasmids; virus vectors such as retroviruses, lentiviruses, adenoviruses, adeno-associated viruses, herpesviruses, and Sendai viruses; and Agrobacterium vectors.

The guide RNA components described above can be easily produced in accordance with known genetic engineering methods. For example, production is possible using PCR, restriction enzyme cleavage, DNA ligation technique, *in vitro* transcription/translation technique, recombinant protein production technique, and the like.

### 2-2. Material (b)

The material (b) is a single-stranded DNA which contains a nucleic acid sequence formed by arranging a 5'-side homology arm sequence, a donor DNA sequence, and a 3'-side homology arm sequence in this order from a 5'-side.

The 5'-side homology arm sequence is a homologous sequence of one nucleic acid sequence outside the genome editing target region, and the 3'-side homology arm sequence is a homologous sequence of the other nucleic acid sequence outside the genome editing target region.

In the design based on the sense strand of the genome target region, the 5'-side homology arm sequence is a homologous sequence of the nucleic acid sequence outside the 5'-side of the genome editing target region sense strand, and the 3'-side homology arm sequence is a homologous sequence of the nucleic acid sequence outside the 3'-side of the genome editing target region sense strand.

In the design based on the antisense strand of the genome target region, the 5'-side homology arm sequence is a homologous sequence of the nucleic acid sequence outside the 5'-side of the genome editing target region antisense strand, and the 3'-side homology arm sequence is a homologous sequence of the nucleic acid sequence outside the 3'-side of the genome editing target region antisense strand.

The identity of the 5'-side homology arm sequence with the one nucleic acid sequence outside the genome editing target region is, for example, 80% or more, preferably 85% or more, more preferably 90% or more, further preferably 95% or more, even further preferably 97% or more, even further preferably 98% or more, even further preferably 99% or more, and even further preferably 100%.

The identity of the 3'-side homology arm sequence with the other nucleic acid sequence outside the genome editing target region is, for example, 80% or more, preferably 85% or more, more preferably 90% or more, further preferably 95% or more, even further preferably 97% or more, even further preferably 98% or more, even further preferably 99% or more, and even further preferably 100%.

The length of the 5'-side homology arm sequence is not particularly limited, but is, for example, 10 bases long or more, preferably 20 bases long or more, more preferably 30 bases long or more, further preferably 40 bases long or more, even further preferably 50 bases long or more, even further preferably 60 bases long or more, even further preferably 80 bases long or more, even further preferably 100 bases long or more, even further preferably 110 bases long or more, even further preferably 130 bases long or more, even further preferably 150 bases long or more, even further preferably 200 bases long or more, and even further preferably 250 bases long or more (for example, 260 bases long or more, 270 bases long or more, 280 bases long or more, 290 bases long or more, 300 bases long or more, and 310 bases long or more), and is, for example, 2000 bases long or less, preferably 1000 bases long or less, more preferably 600 bases long or less, further preferably 400 bases long or less, and even further preferably 350 bases long or less. The range of the length of the 5'-side homology arm sequence can be a combination of any of the upper limits and any of the lower limits described above, and is, for example, 10 to 2000 bases long, 10 to 1000 bases long, 10 to 600 bases long, 40 to 600 bases long, 50 to 600 bases long, 60 to 600 bases long, 80 to 600 bases long, 100 to 600 bases long, 110 to 600 bases long, 130 to 600 bases long, 150 to 600 bases long, 200 to 600 bases long, 250 to 600 bases long (for example, 260 to 600 bases long), 200 to 400 bases long, 200 to 350 bases long, and 250 to 350 bases long (for example, 260 to 350 bases long). When the length of the 5'-side homology arm sequence is made relatively long, it is possible to more efficiently perform the intended genome editing.

The length of the 3'-side homology arm sequence is not particularly limited, but is, for example, 10 bases long or more, preferably 20 bases long or more, more preferably 30 bases long or more, further preferably 40 bases long or more, and even further preferably 50 bases long or more, and is, for example, 2000 bases long or less, preferably 1000 bases long or less, more preferably 600 bases long or less, further preferably 400 bases long or less, even further preferably 200 bases long or less, even further preferably 150 bases long or less, even further preferably 100 bases long or less, and even further preferably 80 bases long or less. The range of the length of the 3'-side homology arm sequence can be a combination of any of the upper limits and any of the lower limits described above, and is, for example, 10 to 2000 bases long, 10 to 1000 bases long, 10 to 600 bases long, 10 to 400 bases long, 20 to 400 bases long, 30 to 400 bases long, 30 to 200 bases long, 30 to 150 bases long, 30 to 100 bases long, 40 to 100 bases long, 50 to 100 bases long, and 50 to 80 bases long.

As the donor DNA sequence, it is possible to employ any nucleic acid sequence. The *in vitro* genome editing method described herein replaces the genome editing target region with the donor DNA sequence. As an example, the donor DNA sequence can be a sequence obtained by introducing a mutation (for example, substitution, deletion, addition, or insertion of a base) into the genome editing target region. As another example, the donor DNA sequence can contain an exogenous DNA sequence. Examples of the exogenous DNA sequence include recombinant enzyme recognition sequences (for example, loxP sequence and FRT sequence), recombinant enzymes (for example, Cre and FLP) expression cassettes, drug resistance gene expression cassettes, negative selection marker expression cassettes, and fluorescent protein expression cassettes. In this case, as an example, the donor DNA sequence can be a sequence obtained by introducing an exogenous DNA sequence into the genome editing target region (or a mutant sequence thereof).

It is difficult to introduce mutations (for example, insertion of two loxP sequences) into more than one site in a certain region at one time in conventional methods using a relatively short single-stranded oligo (ssODN) (such as NPL 1), but the present invention makes it possible to introduce such mutations at one time. From this viewpoint, it is possible to particularly take advantage of the present invention in the case where the donor DNA sequence contains two or more exogenous DNA sequences, mutations to the genome editing target region, or the like, preferably in the case where at least two of them are present away from each other by 50 bases long or more (more preferably 100 bases long or more, further preferably 200 bases long or more, and even further preferably 300 bases long or more).

The length of the donor DNA sequence is not particularly limited. The length is, for example, 10 bases long or more, preferably 30 bases long or more, more preferably 60 bases long or more, further preferably 100 bases long or more, even further preferably 150 bases long or more, and even further preferably 200 bases long or more, and, for example, 10000 bases long or less, preferably 5000 bases long or less, more preferably 2000 bases long or less, further preferably 1000 bases long or less, and even further preferably 800 bases long or less. It is possible to particularly take advantage of the present invention in the case where the donor DNA sequence is long.

The identity of the donor DNA sequence with the genome editing target region is not particularly limited. The identity is, for example, 20% or more, preferably 40% or more, more preferably 60% or more, further preferably 70% or more, and even further preferably 80% or more.

The nucleic acid sequence of the single-stranded DNA is usually a nucleic acid sequence composed only of the 5'-side homology arm sequence, the donor DNA sequence, and the 3'-side homology arm sequence with an arrangement in the order of the 5'-side homology arm sequence, the donor DNA sequence, and the 3'-side homology arm sequence from the 5'-side, but is not limited to the above as long as the genome editing efficiency is not impaired. For example, the nucleic acid sequence may be added with an optional sequence.

When the 5'-side of the 5'-side homology arm sequence is added with an optional sequence, the identity of the nucleic acid sequence composed of the 5'-side homology arm sequence and the optional sequence with the one nucleic acid sequence outside the genome editing target region is, for example, 80% or more, preferably 85% or more, more preferably 90% or more, further preferably 95% or more, even further preferably 97% or more, even further preferably 98% or more, even further preferably 99% or more, and even further preferably 100%.

When the 3'-side of the 3'-side homology arm sequence is added with an optional sequence, the identity of the nucleic acid sequence composed of the 3'-side homology arm sequence and the optional sequence with the other nucleic acid sequence outside the genome editing target region is, for example, 80% or more, preferably 85% or more, more preferably 90% or more, further preferably 95% or more, even further preferably 97% or more, even further preferably 98% or more, even further preferably 99% or more, and even further preferably 100%.

Single-stranded DNAs can be easily produced in accordance with known genetic engineering methods. For example, production is possible using PCR, restriction enzyme cleavage, DNA ligation technique, and the like. In addition, easier and more efficient production is possible by preparing a plasmid DNA containing a single-stranded DNA sequence and nicking endonuclease recognition sites at both ends thereof, digesting the plasmid DNA using nicking endonucleases, subjecting the obtained digest to agarose gel electrophoresis, and extracting single-stranded DNA from gel containing a band matching the intended single-stranded DNA, in accordance with a previous report (Yoshimi, K. et al. ssODN-mediated knock-in with CRISPR-Cas for large genomic regions in zygotes. Nat Commun 7, 10431 (2016).).

### 3. Genome Editing Method and Method for Producing Genome Edited Cell or Organism

The method including the step of introducing the materials (a) and (b) described above into a genome editing target cell or a genome editing target organism makes it possible to edit the genome of the cell or the organism, more specifically to replace the genome editing target region with any donor DNA sequence.

The method for introducing the materials (a) and (b) is not particularly limited, and can be selected as appropriate depending on the type of the target cell or organism or on the types of the materials (such as nucleic acid or protein). Examples of the introduction method include electroporation, microinjection, DEAE-dextran treatment, lipofection, nanoparticle mediated transfection, and virus mediated nucleic acid delivery. When the materials (a) and (b) are all nucleic acids (in particular, when the material a is guide RNA and Cas mRNA), easier and more efficient introduction is possible by employing electroporation. Electroporation can be performed in accordance with, for example, a previous report (Japanese Unexamined Patent Application Publication No. 2015-070825) or Examples to be described later.

Means for introducing the materials (a) and (b) is not particularly limited as long as the materials (a) and (b) are all ultimately delivered into the target cell or organism. The materials (a) and (b) can be introduced simultaneously, sequentially, or separately at predetermined intervals.

Since genome editing occurs in the target cell or the target organism a certain period of time after the introduction of the materials (a) and (b), it is possible to obtain, by recovering this cell or organism, a genome edited (that is, the genome editing target region has been replaced with any donor DNA sequence) cell or organism. In the case where the materials (a) and (b) have been introduced into a fertilized egg, it is possible to obtain a genome edited (that is, the genome editing target region has been replaced with any donor DNA sequence) cell or organism by optionally culturing the obtained fertilized egg for a certain period of time, followed by implantation into the uterus of a pseudopregnant female individual to obtain a child born from the individual.

### 4. Genome Editing Kit and Genome Editing Composition

The materials (a) and (b) can be used as a genome editing composition integrally containing them, or can be used as a kit.

The genome editing composition is not particularly limited as long as it contains the materials (a) and (b) described above, and may further optionally contain additional components. The additional components are not particularly limited as long as they are pharmaceutically acceptable components, and examples thereof include bases, carriers, solvents, dispersants, emulsifiers, buffer agents, stabilizers, excipients, binders, binders, disintegrants, lubricants, thickeners, humectants, colorants, perfumes, and chelating agents.

The genome editing kit is not particularly limited as long as it includes the materials (a) and (b) described above separately or integrally, and may optionally include as appropriate e.g. an instrument and an additional reagent necessary for carrying out the *in vitro* genome editing method described herein such as a nucleic acid introducing reagent, a protein introducing reagent, or a buffer solution.

### [Examples]

Hereinafter, the present invention is described in detail based on Examples, but the present invention is not limited by these Examples.

### Example 1. Genome Editing Test 1 (Wild Type Mouse, Serpina3n Gene, Microinjection)

The genome editing target region was exon 4 of the mouse Serpina3a (serine peptidase inhibitor clade A member 3A) gene and its surrounding region. The region was replaced with a sequence obtained by adding loxP sequences to both ends of the region. Specifically, the procedures were in accordance with the scheme illustrated in Fig. 1.

### <Example 1-1. Production of Guide RNA>

The guide RNA was designed using a software tool (crispr.genome-engineering.org). An *in vitro* transcription kit (MEGAshortscript T7 Transcription Kit, manufactured by Life Technologies) was used with a double-stranded DNA (synthesized by Integrated DNA Technologies) as a template to synthesize a guide RNA designed to cleave one end (Serpina3a gRNA-1, the sequence of the target site (target strand) having SEQ ID NO: 2) and a guide RNA designed to cleave the other end (Serpina3a gRNA-2, the sequence of the target site (target strand) having SEQ ID NO: 3) of the genome editing target region (SEQ ID NO: 1) .

### <Example 1-2. Production of Cas9 mRNA>

Synthesis was carried out using an *in vitro* transcription kit (MEGAshortscript T7 Transcription Kit, manufactured by Life Technologies) where the template DNA was a linearized plasmid containing a sequence obtained by adding a poly A tail to the downstream of the Cas9 coding sequence (pCas9-polyA, Addgene ID #72602), and purification was carried out using a purification kit (MEGAClear kit, manufactured by Life Technologies).

### <Example 1-3. Production of Single-Stranded DNA>

A single-stranded DNA was produced in accordance with a previous report (Yoshimi, K. et al. ssODN-mediated knock-in with CRISPR-Cas for large genomic regions in zygotes. Nat Commun 7, 10431 (2016).), the single-stranded DNA being composed of a nucleic acid sequence (SEQ ID NO: 4) formed by arranging the 5'-side homology arm sequence (sequence of about 300 bases long outside the 5'-side of the genome editing target region antisense strand (complementary sequence having SEQ ID NO: 1)), the loxP sequence (reverse direction), the genome editing target region antisense strand sequence (complementary sequence having SEQ ID NO: 1), the loxP sequence (reverse direction), and the 3'-side homology arm sequence (sequence of about 60 bases long outside the 3'-side of the genome editing target region antisense strand (complementary sequence having SEQ ID NO: 1)) in this order from the 5'-side. Fig. 2 illustrates an overview of the production method.

The details are as follows. An artificial gene synthesis service (GeneArt (registered trademark), Thermo Fisher Scientific) was used to synthesize a plasmid DNA containing the nucleic acid sequence of the single-stranded DNA described above and nicking endonuclease recognition sites arranged on both sides of the nucleic acid sequence. The obtained plasmid DNA was digested using nicking endonucleases which recognize both sides of the nucleic acid sequence of the single-stranded DNA, and the obtained digested product was subjected to agarose gel electrophoresis. A band matching the intended single-stranded DNA was cut out, and the intended single-stranded DNA was extracted from the obtained gel using a DNA extraction kit (NucleoSpin (registered trademark) Gel and PCR Clean-up, manufactured by Takara Bio Inc.).

### <Example 1-4. Introduction into Fertilized Egg and Genotyping>

C57BL/6J Jcl female mice were placed in a superovulated state by the injection of pregnant mare serum gonadotropin (manufactured by ASKA Pharmaceutical Co., Ltd.) and human chorionic gonadotropin (manufactured by ASKA Pharmaceutical Co., Ltd.). Pronuclear stage embryos were collected from the obtained superovulated mice, and cultured in KSOM medium (manufactured by Ark Resource). The above-prepared guide RNA (25 ng/µL), Cas9 mRNA (50 ng/µL), and single-stranded DNA (50 ng/µL) were microinjected into the male pronuclei in the embryos using a micromanipulator (manufactured by Narishige). The embryos were cultured, and 2-cell stage embryos were transferred into the uteri of pseudopregnant female mice. Genomic DNA was extracted from the tails of the born mice (F0 mice), and genotyping was performed by PCR using a primer set designed outside the genome editing target region and the sequencing of the obtained PCR fragments.

### <Example 1 Results>

Table 1 at the end presents the results (the row of Mouse C57B6 Serpina3n MI) . As presented in Table 1, with a high efficiency of 16.1% of the born mice, F0 mice were obtained having an allele whose genome editing target region had been replaced with a sequence added with loxP sequences at both ends of the region (specifically, mice for which the intended genome editing was successful).

In addition, as a result of crossing these F0 mice with C57BL/6J Jcl mice and genotyping the obtained F1 mice as well, it was confirmed that the allele subjected to the intended genome editing was capable of germline transmission.

### Example 2. Genome Editing Test 2 (Wild Type Mouse, Tyr Gene, Microinjection)

The genome editing target region was exon 2 of the mouse Tyr (Tyrosinase) gene and its surrounding region. The region was replaced with a sequence obtained by adding loxP sequences to both ends of the region. Specifically, the procedures were the same as those of Example 1 except that the genome editing target region had SEQ ID NO: 5, the guide RNAs used were Tyr gRNA-1 (the sequence of the target site (non-target strand) had SEQ ID NO: 6) and Tyr gRNA-2 (the sequence of the target site (non-target strand) had SEQ ID NO: 7), and the single-stranded DNA used was a single-stranded DNA composed of a nucleic acid sequence (SEQ ID NO: 8) formed by arranging the 5'-side homology arm sequence (sequence of about 300 bases long outside the 5'-side of the genome editing target region antisense strand (complementary sequence having SEQ ID NO: 5)), the loxP sequence (reverse direction), the genome editing target region antisense strand sequence (complementary sequence having SEQ ID NO: 5), the loxP sequence (reverse direction), and the 3'-side homology arm sequence (sequence of about 50 bases long outside the 3'-side of the genome editing target region antisense strand (complementary sequence having SEQ ID NO: 5)) in this order from the 5'-side.

Table 1 at the end presents the results (the row of Mouse C57B6 Tyr MI) . As presented in Table 1, with a high efficiency of 17.6% of the born mice, F0 mice were obtained having an allele whose genome editing target region had been replaced with a sequence added with loxP sequences at both ends of the region (specifically, mice for which the intended genome editing was successful).

In addition, as a result of crossing these F0 mice with C57BL/6J Jcl mice and genotyping the obtained F1 mice as well, it was confirmed that the allele subjected to the intended genome editing was capable of germline transmission.

### Example 3. Genome Editing Test 3 (Wild Type Mouse, Serpina3n Gene, Electroporation)

The guide RNA, Cas9 mRNA, and single-stranded DNA were introduced in the same manner as that of Example 1 except that the microinjection method was replaced with the electroporation method in accordance with a previous report (JP 2015-070825 A).

The conditions of the electroporation method were as follows. An electroporator (NEPA21 Super Electroporator, manufactured by Nepa Gene) was used to introduce guide RNA (100 ng/µL), Cas9 mRNA (400 ng/µL), and single-stranded DNA (40 ng/µL) into pronuclear stage embryos . The conditions of the poring pulse were such that voltage: 225 V, pulse width: 1 to 2.5 ms, pulse interval: 50 ms, count: 4 times, and polarity: +, and the conditions of the first and second transfer pulses were such that voltage: 20 V, pulse width: 50 ms, pulse interval: 50 ms, count: 5 times, and polarity: ±.

Table 1 at the end presents the results (the row of Mouse C57B6 Serpina3n EL). As presented in Table 1, with a high efficiency of 11.1% of the born mice, F0 mice were obtained having an allele whose genome editing target region had been replaced with a sequence added with loxP sequences at both ends of the region (specifically, mice for which the intended genome editing was successful).

Moreover, as a result of crossing these F0 mice with Cre-driver mice (B6.Cg-Tg(CAG-Cre)CZ-MO2Osb, RBRC01828) and analyzing the obtained F1 mice, it was confirmed that a deletion between the loxP sequences occurred in the F1 mice.

### Example 4. Genome Editing Test 4 (Wild Type Mouse, Mct4 Gene, Electroporation)

The genome editing target region was exon 4 of the mouse Mct4 (Monocarboxylate transporter 4) gene and its surrounding region. The region was replaced with a sequence obtained by adding loxP sequences to both ends of the region. Specifically, the procedures were the same as those of Example 3 except that the genome editing target region had SEQ ID NO: 9, the guide RNAs used were Mct4 gRNA-1 (the sequence of the target site (target strand) had SEQ ID NO: 10) and Mct4 gRNA-2 (the sequence of the target site (target strand) had SEQ ID NO: 11), and the single-stranded DNA used was a single-stranded DNA composed of a nucleic acid sequence (SEQ ID NO: 12) formed by arranging the 5'-side homology arm sequence (sequence of about 300 bases long outside the 5'-side of the genome editing target region sense strand (SEQ ID NO: 9)), the loxP sequence, the genome editing target region sense strand sequence (SEQ ID NO: 9), the loxP sequence, and the 3'-side homology arm sequence (sequence of about 50 bases long outside the 3'-side of the genome editing target region sense strand (SEQ ID NO: 9)) in this order from the 5'-side.

Table 1 at the end presents the results (the row of Mouse C57B6 Mct4 EL) . As presented in Table 1, with a high efficiency of 9.5% of the born mice, F0 mice were obtained having an allele whose genome editing target region had been replaced with a sequence added with loxP sequences at both ends of the region (specifically, mice for which the intended genome editing was successful).

### Example 5. Genome Editing Test 5 (Wild Type Rat, Vapb Gene, Electroporation)

The genome editing target region was exon 2 of the rat Vapb (vesicle-associated membrane protein-associated protein B/C) gene and its surrounding region. The region was replaced with a sequence obtained by adding loxP sequences to both ends of a region introduced with a mutation (a mutation which mutates the 56th amino acid (proline) from the N-terminus of Vapb protein to serine: CCC → TCC) into the region. Specifically, the procedures were in accordance with the scheme illustrated in Fig. 3.

### <Example 5-1. Production of Guide RNA>

A guide RNA (Vapb gRNA-1, the sequence of the target site (target strand) had SEQ ID NO: 14) designed to cleave one end and a guide RNA (Vapb gRNA-2, the sequence of the target site (target strand) had SEQ ID NO: 15) designed to cleave the other end of the genome editing target region (SEQ ID NO: 13) were synthesized in the same manner as that of Example 1-1.

### <Example 5-2. Production of Cas9 mRNA>

Production was the same as that of Example 1-2.

### <Example 5-3. Production of Single-Stranded DNA>

A single-stranded DNA was produced in the same manner as that of Example 1-3, the single-stranded DNA being composed of a nucleic acid sequence (SEQ ID NO: 17) formed by arranging the 5'-side homology arm sequence (sequence of about 250 bases long outside the 5'-side of the genome editing target region sense strand (SEQ ID NO: 13)), the loxP sequence, a sequence (SEQ ID NO: 16) formed by mutation of the 178th base from the 5'-end (cytosine) to thymine in the genome editing target region sense strand sequence (SEQ ID NO: 13), the loxP sequence, and the 3'-side homology arm sequence (sequence of about 60 bases long outside the 3'-side of the genome editing target region sense strand (SEQ ID NO: 13)) in this order from the 5'-side.

### <Example 5-4. Introduction into Fertilized Egg and Genotyping>

The procedures were the same as those of Example 3 except that the rats used for collecting fertilized eggs were F344/Jcl female rats, and the medium used for culturing pronuclear stage embryos was m-KRB (modified Krebs-Ringer Bicarbonate) medium (manufactured by Ark Resource).

### <Example 5 Results>

Table 1 at the end presents the results (the row of Rat F344 Vapb EL) . As presented in Table 1, with a high efficiency of 50.0% of the born rats, F0 rats were obtained having an allele whose genome editing target region had been replaced with a sequence obtained by adding loxP sequences to both ends of a region introduced with a mutation (a mutation which mutates the 56th amino acid (proline) from the N-terminus of Vapb protein to serine: CCC → TCC) into the region (specifically, rats for which the intended genome editing was successful).

In addition, as a result of crossing these F0 rats with F344/Jcl rats and genotyping the obtained F1 rats as well, it was confirmed that the allele subjected to the intended genome editing was capable of germline transmission.

### Example 6. Genome Editing Test 6 (Cre Mouse, Serpina3n Gene, Microinjection)

The procedures were the same as those of Example 1 except that the targets for introduction of guide RNA, Cas9 mRNA, and single-stranded DNA used were C57BL/6J Jcl female mouse fertilized eggs fertilized *in vitro* using sperms of Cre-driver male mice (Emx1-cre, RBRC00808: mice expressing Cre protein in cortical neurons and glia).

Table 1 at the end presents the results (the row of Mouse Emx1-Cre Serpina3n MI). As presented in Table 1, with a high efficiency of 12.5% of the born mice, F0 mice were obtained having an allele whose genome editing target region had been replaced with a sequence added with loxP sequences at both ends of the region (specifically, mice for which the intended genome editing was successful).

In addition, as a result of extracting genomic DNA from various tissues of these F0 mice and performing genotyping, it was confirmed that a deletion between the loxP sequences occurred in a brain tissue specific manner.

### Example 7. Genome Editing Test 7 (Cre Mouse, Serpina3n Gene, Electroporation)

The procedures were the same as those of Example 6 except that the guide RNA, Cas9 mRNA, and single-stranded DNA were introduced by the electroporation method.

Table 1 at the end presents the results (the row of Mouse Emx1-Cre Serpina3n EL). As presented in Table 1, with a high efficiency of 18.5% of the born mice, F0 mice were obtained having an allele whose genome editing target region had been replaced with a sequence added with loxP sequences at both ends of the region (specifically, mice for which the intended genome editing was successful).

In addition, as a result of extracting genomic DNA from various tissues of these F0 mice and performing genotyping, it was confirmed that a deletion between the loxP sequences occurred in a brain tissue specific manner.

**[Table 1]**

| Species | Strain | Target gene | Transfer method | Embryos injected (n) | Embryos transferred (%) | Live births (%) | F0 animals | | | | F1 | |
|---|---|---|---|---|---|---|---|---|---|---|---|---|
| | | | | | | | LD (%) | loxP (%) | flox (%) | Conditional (%) | Germline transmission | Cre-loxP |
| Mouse | C57B6 | *Serpina3n* | MI | 371 | 255 (68.7) | 56 (22.0) | 28 (50.0) | 7 (12.5) | 9 (16.1) | 5(8.9) | 4.14 | - |
| | C57B6 | *Tyr* | MI | 222 | 149 (67.1) | 17 (11.4) | 2 (11.8) | 4 (23.5) | 3 (17.6) | - | 2/2 | - |
| | C57B6 | *Serpina3n* | EL | 180 | 160 (88.9) | 18 (11.3) | 9 (50.0) | 2 (11.1) | 2 (11.1) | 1 (5.6) | - | 212 |
| | C57B6 | *Mct4* | EL | 134 | 130 (97.0) | 21 (16.2) | 11 (52.4) | 3 (14.3) | 2 (9.5) | 2 (9.5) | - | |
| Rat | F344 | *Vapb* | EL | 160 | 77 (48.1) | 6 (7.8) | 2 (33.3) | 1 (16.6) | *3 (50.0) | 1 (16.6) | 3/3 | - |
| Mouse | Emx1-cre | *Serpina3n* | MI | 150 | 74 (49.3) | 8 (10.8) | 4 (50.0) | 1 (12.5) | 1 (12.5) | - | - | - |
| | Emx1-cre | *Serpina3n* | EL | 150 | 113 (75.3) | 27 (23.9) | 10 (37.0) | 1 (3.7) | 5 (18.5) | 3 (11.1) | - | **1/1 |

Explanation of Table 1:
"Species" and "Strain" indicate the species and strain of the genome editing target organism.
"Target" indicates the gene name of the genome editing target.
"Transfer methods" indicates the method for introducing guide RNA, Cas9 mRNA, and single-stranded DNA. "MI" indicates the microinjection method, and "EL" indicates the electroporation method.
"Embryos injected" indicates the number of embryos introduced with guide RNA, Cas9 mRNA, and single-stranded DNA.
"Embryos transferred" indicates the number of embryos transferred to the uteri of pseudopregnant female individuals, and the percentage (%) of that number relative to the number of "Embryos injected."
"Live births" indicates the number of individuals born, and the percentage (%) of that number relative to the number of "Embryos transferred."
"LD" indicates the number of individuals having an allele in which a relatively large region including part or all of the genome editing target region had been deleted (large deletion), and the percentage (%) of that number of individuals relative to the number of "Live births" individuals.
"loxP" indicates the number of individuals having an allele introduced with only one loxP site, and the percentage (%) of that number of individuals relative to the number of "Live births" individuals.
"flox" indicates the number of individuals having an allele whose genome editing target region had been replaced with a sequence added with loxP sequences at both ends of the region (specifically, the number of individuals for which the intended genome editing was successful), and the percentage (%) of that number of individuals relative to the number of "Live births" individuals.
"Conditional" indicates the number of individuals whose two alleles were both flox alleles, the total number of individuals having the flox allele and the LD allele, and the percentage (%) of that total number relative to the number of "Live births" individuals.
"Germline transmission" indicates the number of individuals for which the "flox allele" had been confirmed to be capable of germline transmission as a result of also genotyping F1 mice obtained by crossing "flox allele" individuals and wild type individuals.
"Cre-loxP" indicates the number of individuals confirmed to experience a deletion between the loxP sequences in F1 mice or Cre expression tissues as a result of analyzing the Cre expression tissues (Example 7) of F0 mice or the F1 mice obtained by crossing "flox allele" individuals and Cre-driver individuals.
"-" indicates no analysis performed.

In "Live births," in addition to "LD allele," "loxP allele," and "flox allele," individuals were also observed having an allele which experienced deletion of several bases to several tens of bases near the 5'-end and/or near the 3'-end of the genome editing target region.

### [Industrial Applicability]

The present invention makes it possible to easily and efficiently produce a cell and an organism into which exogenous DNA has been introduced on the genome. The present invention is expected to be used not only for research purposes such as the creation of experimental animals, but also in a wide range of industrial fields including e.g. medical fields such as regenerative medicine, agricultural fields such as the creation of crops with useful traits, and industrial fields such as the production of useful substances using microorganisms.

### [Sequence Listing Free Text]

SEQ ID NO: 2
   <223> Serpina3a gRNA-1 target strand
SEQ ID NO: 3
   <223> Serpina3a gRNA-2 target strand
SEQ ID NO: 4
   <223> single-stranded DNA for Serpina3a
SEQ ID NO: 6
   <223> Tyr gRNA-1 non-target strand
SEQ ID NO: 7
   <223> Tyr gRNA-2 non-target strand
SEQ ID NO: 8
   <223> single-stranded DNA for Tyr
SEQ ID NO: 10
   <223> Mct4 gRNA-1 target strand
SEQ ID NO: 11
   <223> Mct4 gRNA-2 target strand
SEQ ID NO: 12
   <223> single-stranded DNA for Mct4
SEQ ID NO: 14
   <223> Vapb gRNA-1 target strand
SEQ ID NO: 15
   <223> Vapb gRNA-2 target strand
SEQ ID NO: 16
   <223> mutated genome editing region
SEQ ID NO: 17
   <223> for single-stranded DNA Vapb

[Sequence Listing]

## Claims

1. An *in vitro* method for producing a genome edited cell, comprising the step of introducing
(a) an artificial nuclease system which cleaves both ends of a genome editing target region, and
(b) a single-stranded DNA which contains a nucleic acid sequence comprising a 5'-side homology arm sequence, a donor DNA sequence, and a 3'-side homology arm sequence in this order from a 5'-side, the 5'-side homology arm sequence being a homologous sequence of one nucleic acid sequence outside the genome editing target region and 110 bases long or more, the donor DNA sequence being 200 bases long or more and 800 bases long or less, and the 3'-side homology arm sequence being a homologous sequence of the other nucleic acid sequence outside the genome editing target region, into a cell by an electroporation method, wherein the cell is not a human zygote.

2. The method according to claim 1, wherein
the artificial nuclease system is a CRISPR/Cas system.

3. The method according to claim 1 or claim 2, wherein
the donor DNA sequence contains two recombinant enzyme recognition sequences, and
a target for introduction of the artificial nuclease system and the single-stranded DNA is a fertilized egg containing a recombinant enzyme expression cassette.

4. The method according to any one of claims 1 to 3, wherein
a length of the 5'-side homology arm sequence is 130 bases long or more.

5. The method according to any one of claims 1 to 3, wherein
a length of the 5'-side homology arm sequence is 260 bases long or more.

6. A genome editing composition for producing a genome edited cell or a genome edited organism, comprising:
(a) an artificial nuclease system which cleaves both ends of a genome editing target region; and
(b) a single-stranded DNA which contains a nucleic acid sequence comprising a 5'-side homology arm sequence, a donor DNA sequence, and a 3'-side homology arm sequence in this order from a 5'-side, the 5'-side homology arm sequence being a homologous sequence of one nucleic acid sequence outside the genome editing target region and 110 bases long or more, the donor DNA sequence being 200 bases long or more and 800 bases long or less, and the 3'-side homology arm sequence being a homologous sequence of the other nucleic acid sequence outside the genome editing target region.

7. A genome editing kit for producing a genome edited cell or a genome edited organism, comprising:
(a) an artificial nuclease system which cleaves both ends of a genome editing target region; and
(b) a single-stranded DNA which contains a nucleic acid sequence comprising a 5'-side homology arm sequence, a donor DNA sequence, and a 3'-side homology arm sequence in this order from a 5'-side, the 5'-side homology arm sequence being a homologous sequence of one nucleic acid sequence outside the genome editing target region and 110 bases long or more, the donor DNA sequence being 200 bases long or more and 800 bases long or less, and the 3'-side homology arm sequence being a homologous sequence of the other nucleic acid sequence outside the genome editing target region.

## Patentansprüche

1. *In-vitro*-Verfahren zum Herstellen einer genomeditierten Zelle, umfassend den Schritt des Einführens
(a) eines künstlichen Nukleasesystems, das beide Enden einer Genomeditierungszielregion spaltet, und
(b) einer einzelsträngigen DNA, die eine Nukleinsäuresequenz enthält, die eine 5'-seitige Homologiearmsequenz, eine Donor-DNA-Sequenz und eine 3'-seitige Homologiearmsequenz in dieser Reihenfolge von einer 5'-Seite aus umfasst, wobei die 5'-seitige Homologiearmsequenz eine homologe Sequenz einer Nukleinsäuresequenz außerhalb der Genomeditierungszielregion und 110 Basen oder länger ist, die Donor-DNA-Sequenz 200 Basen oder länger und 800 Basen oder kürzer ist, und die 3'-seitige Homologiearmsequenz eine homologe Sequenz der anderen Nukleinsäuresequenz außerhalb der Genomeditierungszielregion ist, in eine Zelle durch ein Elektroporationsverfahren, wobei die Zelle keine menschliche Zygote ist.

2. Verfahren nach Anspruch 1, wobei
das künstliche Nukleasesystem ein CRISPR/Cas-System ist.

3. Verfahren nach Anspruch 1 oder 2, wobei
die Donor-DNA-Sequenz zwei rekombinante Enzymerkennungssequenzen enthält, und
ein Ziel für die Einführung des künstlichen Nukleasesystems und der einzelsträngigen DNA eine befruchtete Eizelle ist, die eine Expressionskassette für ein rekombinantes Enzym enthält.

4. Verfahren nach einem der Ansprüche 1 bis 3, wobei
eine Länge der 5'-seitigen Homologiearmsequenz 130 Basen oder länger ist.

5. Verfahren nach einem der Ansprüche 1 bis 3, wobei
eine Länge der 5'-seitigen Homologiearmsequenz 260 Basen oder länger ist.

6. Genomeditierungszusammensetzung zum Herstellen einer genomeditierten Zelle oder eines genomeditierten Organismus, umfassend:
(a) ein künstliches Nukleasesystem, das beide Enden einer Genomeditierungszielregion spaltet, und
(b) eine einzelsträngige DNA, die eine Nukleinsäuresequenz enthält, die eine 5'-seitige Homologiearmsequenz, eine Donor-DNA-Sequenz und eine 3'-seitige Homologiearmsequenz in dieser Reihenfolge von einer 5'-Seite aus umfasst, wobei die 5'-seitige Homologiearmsequenz eine homologe Sequenz einer Nukleinsäuresequenz außerhalb der Genomeditierungszielregion und 110 Basen oder länger ist, die Donor-DNA-Sequenz 200 Basen oder länger und 800 Basen oder kürzer ist, und die 3'-seitige Homologiearmsequenz eine homologe Sequenz der anderen Nukleinsäuresequenz außerhalb der Genomeditierungszielregion ist.

7. Genomeditierungskit zum Produzieren einer genomeditierten Zelle oder eines genomeditierten Organismus, umfassend:
(a) ein künstliches Nukleasesystem, das beide Enden einer Genomeditierungszielregion spaltet, und
(b) eine einzelsträngige DNA, die eine Nukleinsäuresequenz enthält, die eine 5'-seitige Homologiearmsequenz, eine Donor-DNA-Sequenz und eine 3'-seitige Homologiearmsequenz in dieser Reihenfolge von einer 5'-Seite aus umfasst, wobei die 5'-seitige Homologiearmsequenz eine homologe Sequenz einer Nukleinsäuresequenz außerhalb der Genomeditierungszielregion und 110 Basen oder länger ist, die Donor-DNA-Sequenz 200 Basen oder länger und 800 Basen oder kürzer ist, und die 3'-seitige Homologiearmsequenz eine homologe Sequenz der anderen Nukleinsäuresequenz außerhalb der Genomeditierungszielregion ist.

## Revendications

1. Procédé *in vitro* de production d'une cellule à génome édité, comprenant l'étape d'introduction
(a) d'un système de nucléase artificielle qui clive les deux extrémités d'une région cible d'édition de génome, et
(b) d'un ADN simple brin contenant une séquence d'acide nucléique comprenant une séquence de bras d'homologie côté 5', une séquence d'ADN donneur, et une séquence de bras d'homologie côté 3', dans cet ordre à partir d'un côté 5', la séquence de bras d'homologie côté 5' étant une séquence homologue d'une séquence d'acide nucléique extérieure à la région cible d'édition de génome et d'une longueur supérieure ou égale à 110 bases, la séquence d'ADN donneur étant d'une longueur supérieure ou égale à 200 bases et inférieure ou égale à 800 bases, et la séquence de bras d'homologie côté 3' étant une séquence homologue de l'autre séquence d'acide nucléique extérieure à la région cible d'édition de génome, dans une cellule par un procédé d'électropora-tion, dans lequel la cellule n'est pas un zygote humain.

2. Procédé selon la revendication 1, dans lequel
le système de nucléase artificielle est un système CRISPR/Cas.

3. Procédé selon la revendication 1 ou la revendication 2, dans lequel
la séquence d'ADN donneur contient deux séquences de reconnaissance d'enzyme recombinante, et
une cible pour l'introduction du système de nucléase artificielle et de l'ADN simple brin est un oeuf fécondé contenant une cassette d'expression d'enzyme recombinante.

4. Procédé selon l'une quelconque des revendications 1 à 3, dans lequel
une longueur de la séquence de bras d'homologie côté 5' est supérieure ou égale à 130 bases.

5. Procédé selon l'une quelconque des revendications 1 à 3, dans lequel
une longueur de la séquence de bras d'homologie côté 5' est supérieure ou égale à 260 bases.

6. Composition d'édition de génome pour la production d'une cellule à génome édité ou d'un organisme à génome édité, comprenant :
(a) un système de nucléase artificielle qui clive les deux extrémités d'une région cible d'édition de génome ; et
(b) un ADN simple brin contenant une séquence d'acide nucléique comprenant une séquence de bras d'homologie côté 5', une séquence d'ADN donneur, et une séquence de bras d'homologie côté 3', dans cet ordre à partir du côté 5', la séquence de bras d'homologie côté 5' étant une séquence homologue d'une séquence d'acide nucléique extérieure à la région cible d'édition de génome et d'une longueur supérieure ou égale à 110 bases, la séquence d'ADN donneur étant d'une longueur supérieure ou égale à 200 bases et inférieure ou égale à 800 bases, et la séquence de bras d'homologie côté 3' étant une séquence homologue de l'autre séquence d'acide nucléique extérieure à la région cible d'édition de génome.

7. Kit d'édition de génome pour la production d'une cellule à génome édité ou d'un organisme à génome édité, comprenant :
(a) un système de nucléase artificielle qui clive les deux extrémités d'une région cible d'édition de génome ; et
(b) un ADN simple brin contenant une séquence d'acide nucléique comprenant une séquence de bras d'homologie côté 5', une séquence d'ADN donneur, et une séquence de bras d'homologie côté 3', dans cet ordre à partir du côté 5', la séquence de bras d'homologie côté 5' étant une séquence homologue d'une séquence d'acide nucléique extérieure à la région cible d'édition de génome et d'une longueur supérieure ou égale à 110 bases, la séquence d'ADN donneur étant d'une longueur supérieure ou égale à 200 bases et inférieure ou égale à 800 bases, et la séquence de bras d'homologie côté 3' étant une séquence homologue de l'autre séquence d'acide nucléique extérieure à la région cible d'édition de génome.
